# EUROPEAN PATENT SPECIFICATION

(11) **EP 2 531 243 B1**
(45) Date of publication and mention of the grant of the patent: **26.08.2015**
(21) Application number: 11702862.1
(22) Date of filing: 01.02.2011
(51) Int. Cl.: A61M 5/50, A61M 5/32

(54) **MEDICAL NEEDLE SAFETY DEVICE**
MEDIZNISCHE NADELSCHUTZVORRICHTUNG
DISPOSITIF DE SÉCURITÉ D'AIGUILLE MÉDICALE

(30) Priority: 01.02.2010 GB 201001506; 18.10.2010 GB 201017490
(43) Date of publication of application: 12.12.2012
(73) Proprietor: Liversidge, Barry Peter, Colchester, Essex CO4 5PE (GB)
(72) Inventor: Liversidge, Barry Peter, Colchester, Essex CO4 5PE (GB)
(74) Representative: Sanderson & Co.
(86) International application number: PCT/GB2011/050169
(87) International publication number: WO 2011/092524

(56) References cited:
- WO-A1-2009/081133
- US-A1- 2005 277 895
- US-A1- 2009 227 956

## Description

This invention relates to a safety device for use with a medical needle having a sharp tip, to confer passive protection to that needle. The invention also relates to a safety needle assembly comprising such a safety device together with a medical needle and also to an injection device incorporating such a safety needle assembly. An example of prior art medical needle safety device is disclosed in US 2009/227 956 A1.

The invention comprises an improvement to and modification of the needle safety devices described and claimed in my co-pending International Patent Application No. PCT/GB2011/050159, filed on 1 st February 2011, though the invention is applicable to other types of needle safety devices as well. In said application, there is described a medical needle passive safety device having a needle mount for directly or indirectly supporting a needle, a needle shielding sleeve for surrounding a supported needle and arranged coaxially with the mount so that a force applied to the sleeve slides the sleeve from a needle shielding position towards a non-shielding position. An abutment surface and a sliding surface are provided on one of the sleeve and mount and a radially deformable resilient finger is provided on the other of the sleeve and mount. When the finger is in a substantially undeformed condition, the finger blocks sliding movement of the sleeve to a non-shielding position but there is a control member initially at a set position to deform the finger radially outwardly on initial sliding movement of the sleeve to a non-shielding position so that the sleeve may continue sliding to the non-shielding position. The control member is displaced from its set position in the course of the sliding movement of the sleeve so that on subsequent return of the sleeve to its shielding position, the finger returns to its substantially undeformed condition; thereafter the finger blocks movement of the sleeve towards its non-shielding position.

With such a needle safety device, it is possible for the control member to move from its set position so that use of the device is impossible as the finger blocks movement of the sleeve to a non-shielding position. This may happen during handling of the safety device after manufacture and before the device is about to be used to perform an injection. Moreover, accidental handling of the device in preparation for its use may move the sleeve from its shielding position partway towards its non-shielding position so displacing the control member from its set position; upon the sleeve returning to its shielding position it may thereafter not be possible to use the device in the performance of an injection as again the finger will block the movement of the sleeve.

In an attempt to address the problems discussed above of safety devices as discussed above, this invention provides a medical needle safety device as recited in claim 1 having a mount for a medical needle, a sleeve for shielding a mounted needle and movable from an initial shielding position to a non-shielding position, a finger provided on one of the sleeve and mount and resiliently deformable outwardly by movement of the sleeve from its initial shielding position to a non-shielding position, and a package component for the safety device which overlies at least a part of the finger to prevent outward deformation of the finger until the package component is removed from the safety device.

It will be appreciated that with the safety device of this invention, the package component prevents outward deformation of the finger until that component has been removed from the safety device. As such, the fingers will continue to block movement of the sleeve towards a non-shielding position and such movement will only be possible once the package component has been removed from the safety device.

In a preferred form of the invention, the sleeve is slidably arranged coaxially with the mount so that a force applied to the sleeve moves the sleeve from an initial needle shielding position to a non-shielding position whereat at least the tip of a supported needle is exposed beyond the sleeve; an abutment surface and a sliding surface is provided on the other of the sleeve and mount; and the finger has a part in radial alignment with the abutment surface to block movement of the sleeve from its needle shielding position. In such an arrangement, there may be a control member arranged coaxially with the sleeve and mount and slidably displaceable from a set position with respect thereto.

In one embodiment the package component is substantially tubular and is provided with an internal rib which overlies the finger to prevent the outward deformation thereof. Alternatively, the package component may closely surround the sleeve to prevent outward deformation of the finger.

With either embodiment, as the package component prevents outward deformation of the finger until the package component has been removed, the fingers themselves may resist displacement of the control member from its set position, so preventing inadvertent activation of the device and ensuring the device is available for use when the package component has been removed.

In the case of a tubular package component, it may have a closed end and an open end, the safety device being insertable into the package component and removable therefrom through the open end of the component. A sealing member may be arranged to close the open end of the package component and so enclose the safety device there within. Alternatively, a removable cap may be provided for the open end of the package component.

The safety device may be associated with a medical needle having a hub received in the mount, and the sleeve being disposed in a shielding position conferring protection on the needle. Alternatively, the safety device may be associated with a single-use syringe having a needle permanently attached thereto, the syringe serving as the needle mount of the safety device and the sleeve is arranged to slide on the syringe.

In the case of a safety device associated with a single use syringe, a needle cover may be provided for the needle, the cover sealing the sharp end of the needle. The package component may include a structure for stripping the needle cover from the needle, on removing the package component from the safety device.

The safety device may have a plurality of fingers spaced around said one of the sleeve and mount, the fingers being resiliently deformable outwardly by sliding axial movement of the sleeve. Typically, a pair of opposed fingers may be provided though other numbers of fingers, such as three disposed at 120° or four arranged mutually at right angles may be provided. In such an arrangement, the resilient outward deformation of the fingers by the sliding axial movement of the sleeve stores energy for returning the sleeve to a shielding position.

By way of example only, two specific embodiments of this invention will now be described in detail, reference being made to the accompanying drawings in which:-
Figure 1 is a diagrammatic axial section through a safety device similar to those of said earlier application but with several of the components thereof shown in outline or omitted altogether for the sake of clarity, and having a package component fitted thereto;
Figure 2 is a view similar to that of Figure 1 but with the package component removed from the safety device;
Figure 3 shows the safety device of Figure 2 in the course of movement of the sleeve from its initial shielding position to its non-shielding position.
Figure 4 is a diagrammatic isometric view of a syringe and a first safety device of the invention described and claimed in said co-pending application, with the device shown in an exploded form, but not showing the package component of the present invention;
Figure 5 is a cross-section through the device of Figure 4, in its initial setting with the sleeve in its initial shielding position;
Figure 6 is similar to Figure 5 but showing movement of the sleeve from the position of Figure 5, and showing the radially outward movement of the fingers;
Figure 7 is similar to Figure 6 but showing the sleeve moved to the non-shielding position;
Figure 8 shows the sleeve returned to and blocked in a needle shielding position;
Figure 9 is a diagrammatic isometric view of a syringe and a second safety device of the invention described and claimed in said co-pending application, with the device shown in an exploded form but not showing the package component of the present invention;
Figure 10 shows the sleeve of the device of Figure 9, part way through being slid from its initial shielding position to its non-shielding position; and
Figures 11 and 12 show a safety device of Figures 4 to 8 or of Figures 9 and 10, arranged as a self-contained package including a needle, ready for fitting to a syringe.

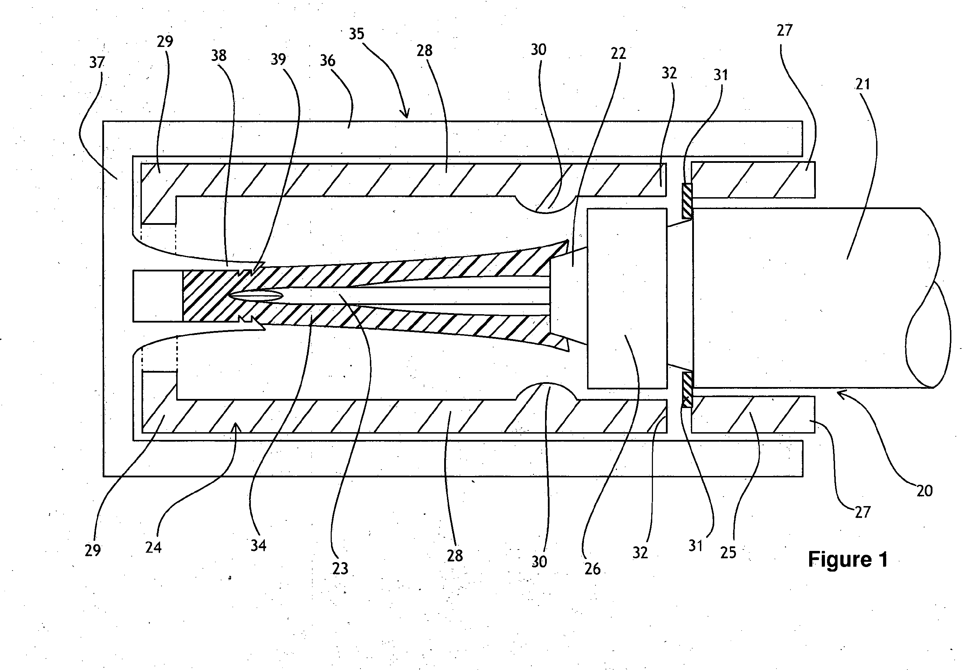

Referring initially to Figures 1 to 3, there is shown diagrammatically a first embodiment of packaged medical needle safety device of this invention. The safety device will be described in more detail hereinafter and in Figures 1 to 3, that device is shown only in outline, for an understanding of the invention.

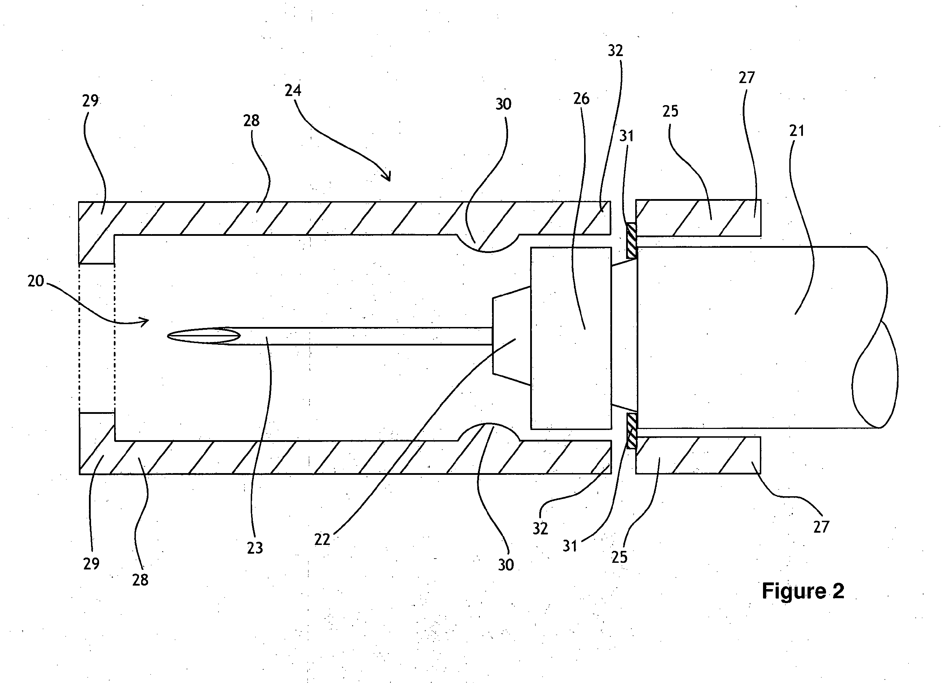

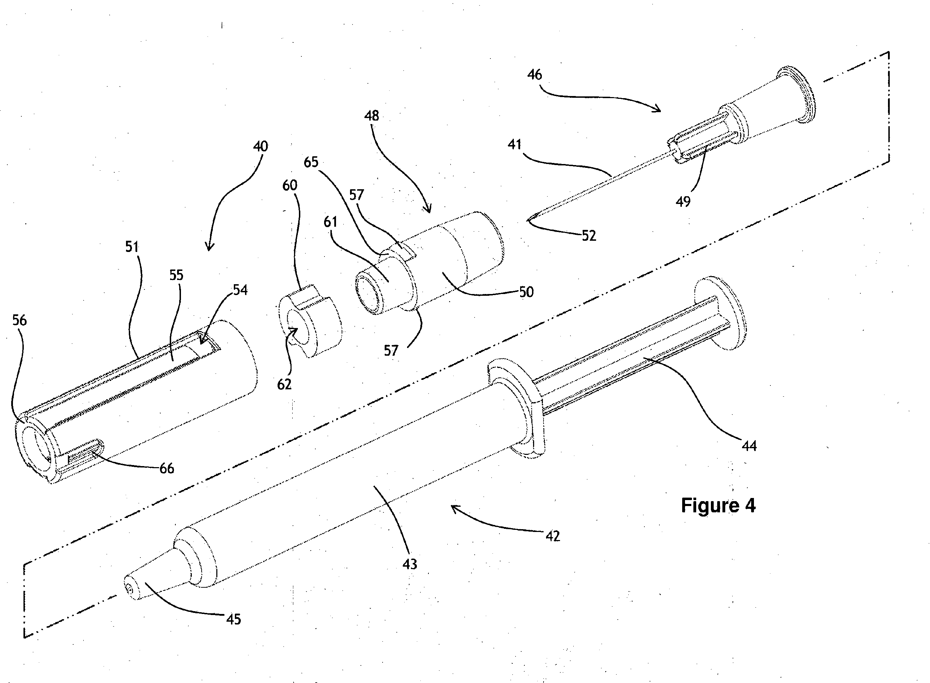

In Figures 1 to 3, a syringe 20 (shown only in part) has a barrel 21 with a nose 22 at its forward end and which has a medical needle 23 permanently staked therein. The syringe 20 typically is pre-filled with a drug which may be expelled by depressing a plunger (not shown) projecting rearwardly from the barrel of the syringe and once used, the entire syringe and needle are disposed of in a safe manner. Protection against needle-stick injuries, both before and after use of the syringe, is afforded by means of a passive safety device 24 mounted directly on the syringe barrel 21. The syringe barrel thus serves as a mount for the safety device, which includes a sleeve 25 slidable rearwardly from a shielding position (shown in Figures 1 and 2) where the sleeve surrounds the needle, to a non-shielding position. The safety device includes a control member 26 mounted on the nose 22 of the syringe and movable from the position shown in Figures 1 and 2 forwardly with respect to the barrel, to allow blocking of the sleeve following the performance of a medical procedure. The operation of the safety device, including a spring arrangement for urging the sleeve forwardly to its protecting position, will be described in more detail below, with reference to Figures 4 to 8.

The sleeve 25 has a rearward part 27 which is slidably carried on the syringe barrel 21 and has a pair of diametrically opposed apertures in which are provided fingers 28 connected to the sleeve at the forward end 29 thereof. The fingers are resiliently deformable radially outwardly from their position shown in Figure 1 as will be described below. Each finger has an inwardly directed protrusion 30 which, in the setting of Figure 1, is disposed closely adjacent but forwardly of the control member 26. A pair of opposed lugs 31 upstand from the junction of the syringe barrel 21 with the nose 22 and are received in the apertures of the sleeve 25. The lugs serve to prevent the sleeve 25 sliding forwardly off the syringe barrel and also to prevent rearward movement of the sleeve when the control member is displaced forwardly from its position shown in Figures 1 and 2 to be located forward of the protrusions 30, whereby the rearward ends 32 of the fingers 28 will abut the lugs if a rearwardly directed force is applied to the sleeve.

A soft needle cover 34 is fitted to the needle 23 and seals against the nose 22, the sharp end of the needle penetrating the material of the needle cover so that the sharp end of the needle is sealed, to prevent leakage of liquid drug out of the needle.

A tubular component 35 is fitted to the safety device 24 so as wholly to surround that device prior to the use thereof. Thus, the tubular component has an outer cylindrical wall 36 and a closed front wall 37, the rear end of the cylindrical wall being open to allow the insertion of the safety device thereinto and through which the safety device may be removed from the component. Internally, a structure 38 upstands centrally from the front wall 37 that structure being provided with inwardly directed barbs 39 which bite into the soft needle cover 34, so that on pulling the tubular component away from the syringe and safety device, the soft needle cover is stripped off the needle and remains within the component.

Though not shown in the drawings, a sealing member may be provided between the tubular component 35 and the syringe barrel 21 in order that the safety device may be maintained in sterile conditions. Further, that sealing member may also provide a tamper-evident seal, in that if the seal is broken, then the syringe should not be used for performing a medical procedure.

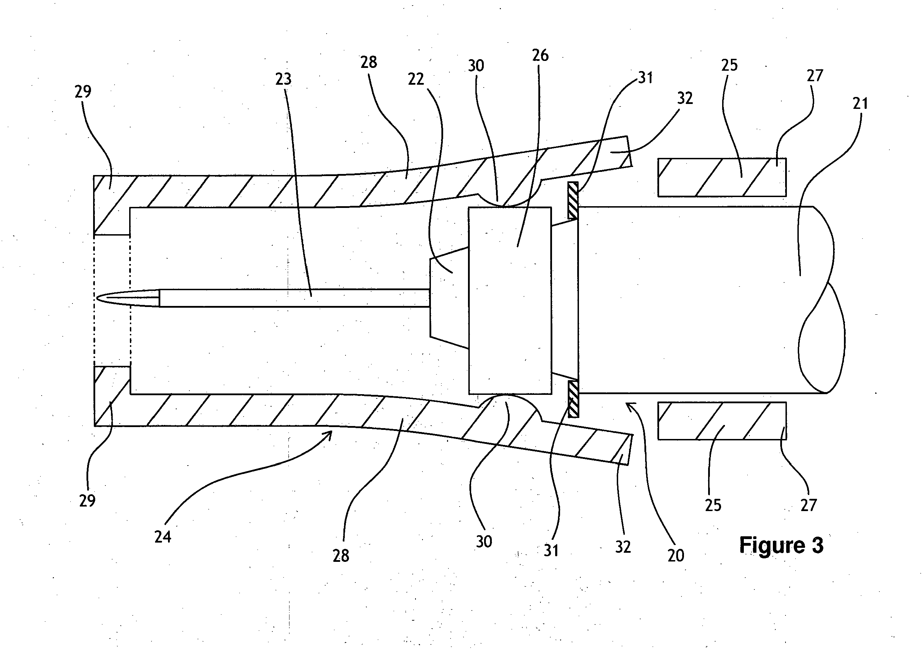

In use, the seal (if one is provided) holding the tubular component 35 to the syringe barrel 21 should be broken so allowing the tubular component to be pulled away from the safety device 24. This simultaneously strips the soft needle cover 34 away from the needle 23, as shown in Figure 2, leaving the device ready to perform an injection. Holding the syringe barrel 21, the forward end of the sleeve 25 is presented to an injection site and on pushing the syringe forwardly so that the needle penetrates that site, the fingers 28 are deformed radially outwardly by the action of the control member 26 on the protrusions 30 (Figure 3). The rearward ends 32 of the fingers are thus moved radially outwardly so as to clear the lugs 31, and this allows the syringe and needle to continue forward movement relative to the sleeve, which remains stationary in contact with the injection site. The radially outward deformation of the fingers stores energy which serves to urge the sleeve forwardly with respect to the syringe.

On completion of the injection, the syringe and needle is pulled away from the injection site while the forward end of the sleeve remains in contact therewith and thus in effect moves forwardly with respect to the syringe to a needle shielding position. Frictional interengagement between the sleeve 25 and the control member 26 displaces the control member forwardly within the sleeve so that on return of the sleeve to a needle shielding position, subsequent rearward movement thereof is blocked by the rearward ends 32 of the fingers 28 engaging the lugs 31.

It will be appreciated that prior to the removal of the tubular component 35 from the safety device, the outward deformation of the fingers 28 is resisted by the internal wall of the tubular component. Consequently, the sleeve 25 cannot be displaced rearwardly with respect to the syringe and needle until the tubular component has been removed. In a practical embodiment, this also serves to prevent the blocking of the rearward movement of the sleeve with respect to the syringe, prior to use of the device to perform an injection.

Figures 1 to 3 diagrammatically show the arrangement of the safety device for a pre-filled syringe and the above description of those Figures is an outline of the action thereof. That action will now be described in more detail with reference to Figures 4 to 8, showing a safety device for a needle provided with a hub for attachment to a conventional syringe, as described and claimed in co-pending International Application No. PCT/GB2011/050159.

The safety device 40 of Figures 4 to 8 is arranged for shielding a medical needle 41 of a conventional form, intended for mounting on a conventional syringe 42. The syringe has a cylindrical body 43 defining a cylindrical chamber for a liquid medicament, there being a plunger 44 fitted with a piston (not shown) at its forward end to expel medicament out of the nose 45 of the syringe. That nose has an external surface formed as a conventional Luer slip taper, for the connection thereto of the needle hub 46 with an internal Luer slip taper. This arrangement is all entirely conventional and will not be described in further detail here.

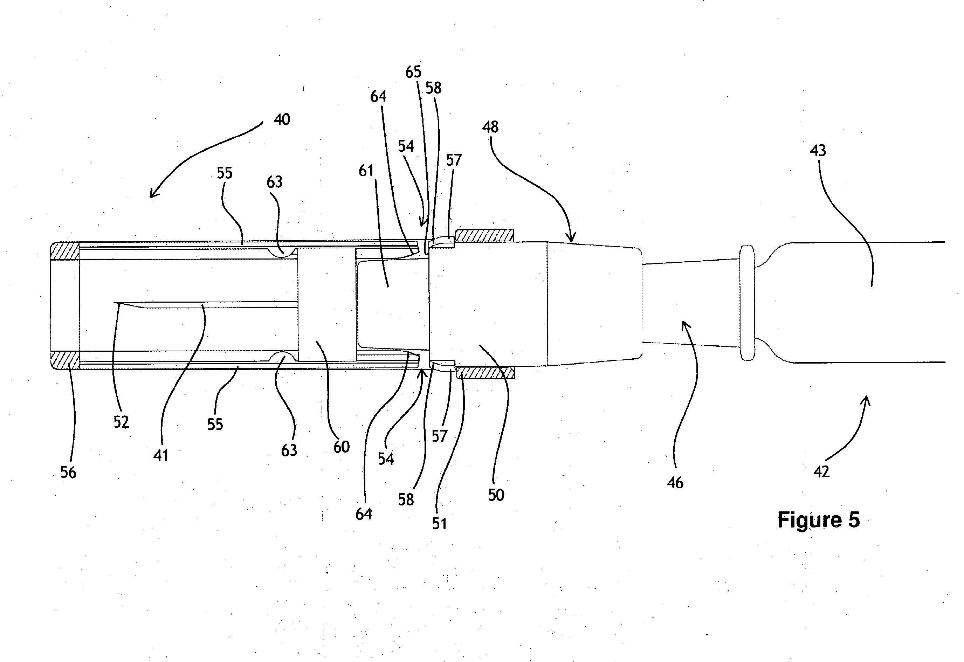

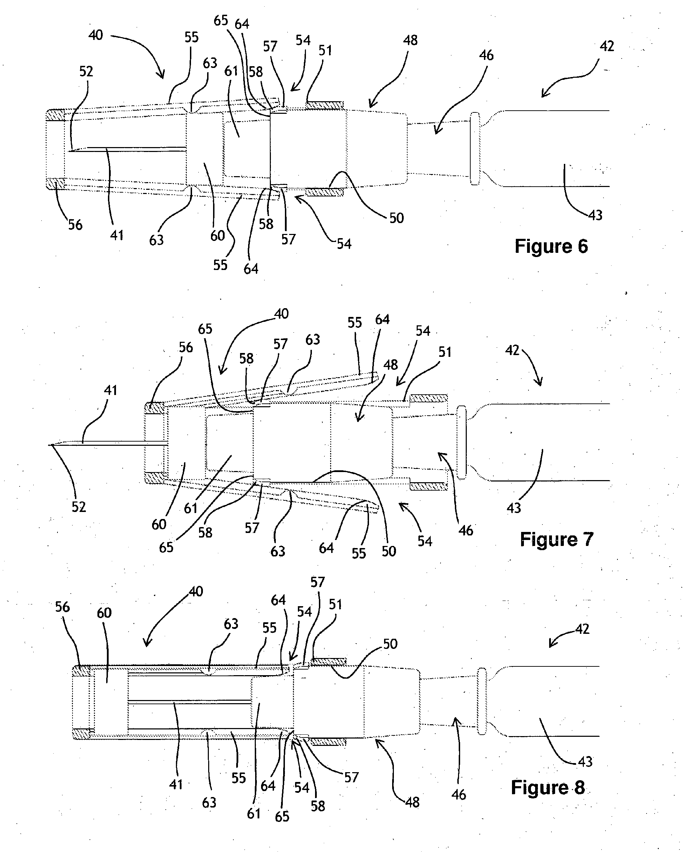

The safety device 40 has a mount 48 provided with a bore which is adapted to be a close push-fit on ribbed section 49 of the needle hub 46, possibly slightly deforming the ribs to ensure there is sufficient friction between the hub and the mount. The mount has a cylindrical sliding surface 50 on which is supported a sleeve 51 arranged for axial sliding movement with respect to the mount and so also with respect to the needle. The initial needle-shielding position is shown in Figure 5, and the sleeve may slide rearwardly to a non-shielding position shown in Figure 7, where part of the needle back from its sharp tip 52 is exposed, so that a medical procedure may be performed. The sliding movement of the sleeve may take place as a part of that procedure, such as performing an injection.

The sleeve has a pair of opposed elongate apertures 54 within which are formed respective fingers 55 connected to the main part of the sleeve at the forward end 56 thereof, for serving as leaf springs. Though two such apertures each having a respective finger are shown, other numbers of apertures of fingers could be employed, ranging from a single aperture and finger up to three or more apertures and fingers. Each finger 55 is resiliently deformable radially outwardly, as will be apparent from the following description of the device.

The mount 48 includes a pair of opposed lugs 57 formed at the forward end of the sliding surface 50 of the mount, which lugs locate in the apertures 54 of the sleeve and serve to prevent removal of the sleeve from the mount, once fitted thereon and the sleeve is in its initial position shown in Figure 5. The forwardly directed surface 58 of each lug is of arcuate form, so as to facilitate fitting of the sleeve to the mount and also to provide a sliding surface for the associated finger, when the sleeve slides with respect to the mount.

A control member 60 is disposed within the sleeve 51 and in the initial setting of the device is disposed against a boss 61 formed at the forward end of the mount 48. The control member has a sufficiently large bore 62 for the needle 41 to pass easily therethrough during assembly but the bore is smaller than the diameter of the boss 61 such that the control member will bear on that boss 61 as the sleeve moves rearwardly, in the course of a procedure. Each finger 55 is formed with an opposed pair of inwardly directed protrusions 63 engageable with the external surface of the control member and disposed closely adjacent the control member in the initial setting of the device as shown in Figure 5. Each finger has a central rib which runs on the sliding surface 50 of the mount as the sleeve moves rearwardly with respect thereto. The rearward end 64 of each finger is formed for engagement with the abutment surface 65 between the sliding surface 50 and boss 61 of the mount when the finger is in an undeformed condition at the initial setting of the device. As shown in the drawings, the free end of each finger may be raked slightly and the abutment surface 65 may be dished such that on the rearward end 64 of the finger engaging the abutment surface, the finger is encouraged to move deeper into engagement.

At the forward end 56 of the sleeve, there are formed two diametrically-opposed windows 66, on a line perpendicular to that extending between the fingers 55. When the sleeve has moved fully rearwardly, so that the control member has been pushed to the forward end 56 of the sleeve, the control member will be visible through those windows 66 so as to serve as an indicator that the device has been used and now has the sleeve locked in the shielding position. This indication may be enhanced by making the control member of a brightly coloured material contrasting with the material of the sleeve.

In this embodiment, each of the mount, sleeve and control member is made of moulded plastics material. The fingers 55 are resiliently deformable radially outwardly by flexing of those fingers but in the initial position shown in Figures 4 and 5, the fingers are in an undeformed (or unstressed) condition. Thus, the device may be stored in that condition without the fingers suffering from a loss of resilience, which otherwise would occur through storage when stressed.

The operation of the safety device 40 described above will now be explained, following the fitting of the device to a needle 41. This action may be performed either before or after the needle hub 46 has been fitted to the Luer slip connector at the forward end of the syringe 42. The initial setting of the device is shown in Figure 5, with the sleeve 51 in its forward shielding position with respect to the needle so as to confer protection thereto. In this setting, the fingers 55 extend substantially parallel to the axis of the sleeve and mount and are in an undeformed as-moulded condition, as mentioned above. The control member 60 is disposed adjacent the boss 61 with the protrusions 63 of the fingers 55 adjacent the forward face of the control member. The sleeve is held against forward movement with respect to the mount 48 by virtue of the engagement of the rear end of each aperture 54 with the respective lugs 57.

The syringe, needle and device are advanced to the injection site and the forward end 56 of the sleeve is pressed against the skin. This moves the sleeve rearwardly with respect to the mount 48 so that the protrusions 63 move up on to the outer surface of the control member 60, which remains stationary with respect to the mount. As shown in Figure 6 this resiliently flexes the fingers radially outwardly so that the rearward ends 64 thereof are lifted clear of the abutment surface 65 and are brought to bear on sliding surfaces 58 of the lugs 57 (Figure 6). Continued forward movement of the syringe moves the sleeve further rearwardly with respect to mount 48 with the inner surfaces of the fingers sliding on the lugs 57 and so being deflected further in the radially outward direction. Eventually, the position is reached where the control member 60 is located at the forward end of the sleeve 51 and the needle projects fully from the forward end of the sleeve (Figure 7). The fingers are deflected to their greatest extent, storing energy and serving as springs urging the sleeve back to a protecting position.

When in the position of Figure 7, the injection may be performed by pressing on the plunger 44 so as to dispense the liquid drug out of the syringe through the needle 41. Then, on moving the syringe away from the skin, the axial force exerted on the sleeve by the resilient fingers 55 moves the sleeve forwardly with respect to the mount while the control member 60 remains at the forward end of the sleeve, maintained there for example by friction. Eventually, the position shown in Figure 8 is reached where the fingers 55 have returned to their original undeformed condition, free of the mount 48 but the control member 60 remains at the forward end 56 of the sleeve. The sleeve is thus once more in the shielding position with the needle surrounded by the sleeve. If now an attempt is made to move the sleeve rearwardly once more, the rearward ends 64 of the fingers 55 will engage the abutment surface 65 of the mount 48 and in view of the respective profiles of the ends of the fingers and that abutment surface, increased rearward pressure on the sleeve will serve to strengthen the interengagement of the fingers with the abutment surface, so blocking rearward movement of the sleeve.

When in the blocked setting of Figure 8, the control member will be visible through the windows 66, so showing that the device has been used and must be discarded. Conveniently, this is done by placing the entire assembly of syringe, needle and device in a suitable sharps container. The indication that the device has been used may be enhanced by brightly colouring the control member so enhancing the visibility thereof through the windows 66.

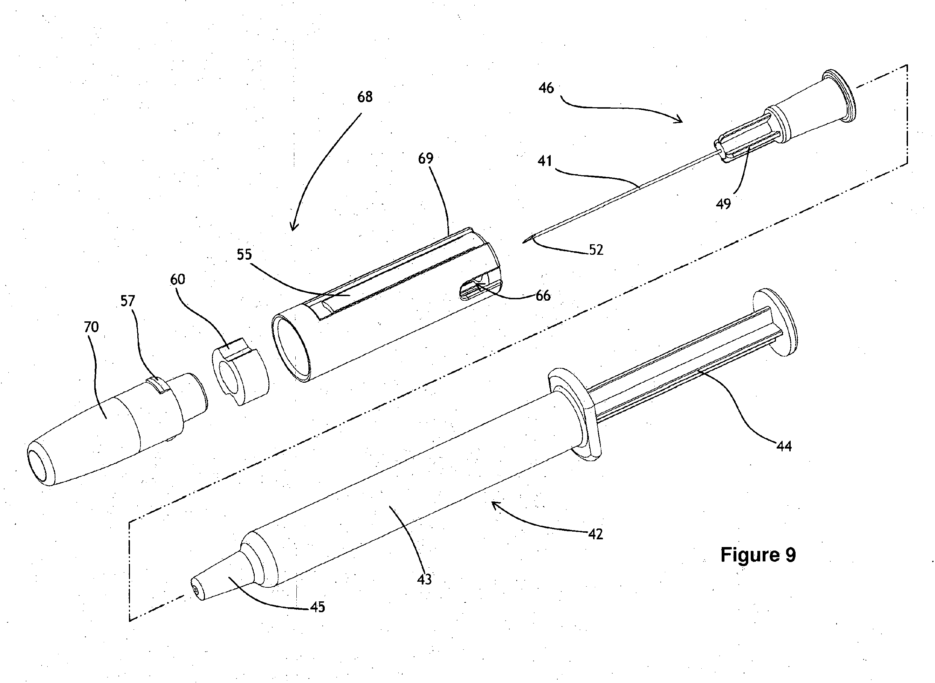

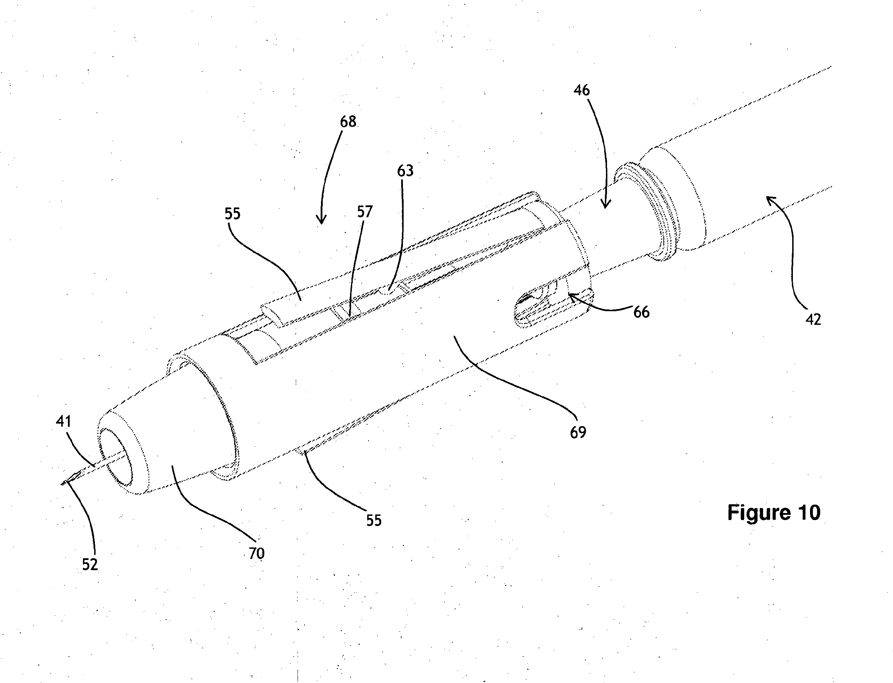

Referring now to Figures 9 and 10, there is shown a second safety device 68 which operates on broadly the same principles as the first safely device 40 (Figures 4 to 8) and insofar as is possible, like parts are indicated by like reference characters and will not be described again here. As before, the syringe 42 and the needle together with its needle hub are essentially conventional. In the second safety device 68, the sleeve 51 of the first device is adapted for mounting directly on the needle hub 46 so as to serve as the mount 69 for the second device. The mount 48 of the first device serves as the sleeve 70 for the second device and so is movable from a fully shielding position rearwardly with respect to the needle to a non-shielding position at which an injection may be performed. Subsequently, the sleeve 70 moves forwardly to return to its shielding position under the action of the fingers 55 which were resiliently deformed radially outwardly in the course of the sleeve 70 moving rearwardly, as shown in Figure 10.

The configuration of the fingers 55, the control member 60, the lugs 57 and the windows 66 are all as described above with reference to the first safety device and have the same functionality. The operation of the second device and the sequence thereof will not therefore be described again here. Suffice it to say that in the first device, the mount 48 is carried on the needle hub 46 and the sleeve 51 slides rearwardly over that mount 48. With the second device, the sleeve 51 (of the first device) serves as the mount 69 and is carried on the needle hub; the mount 48 (of the first device) serves as the sleeve 70 and slides rearwardly within the mount 69.

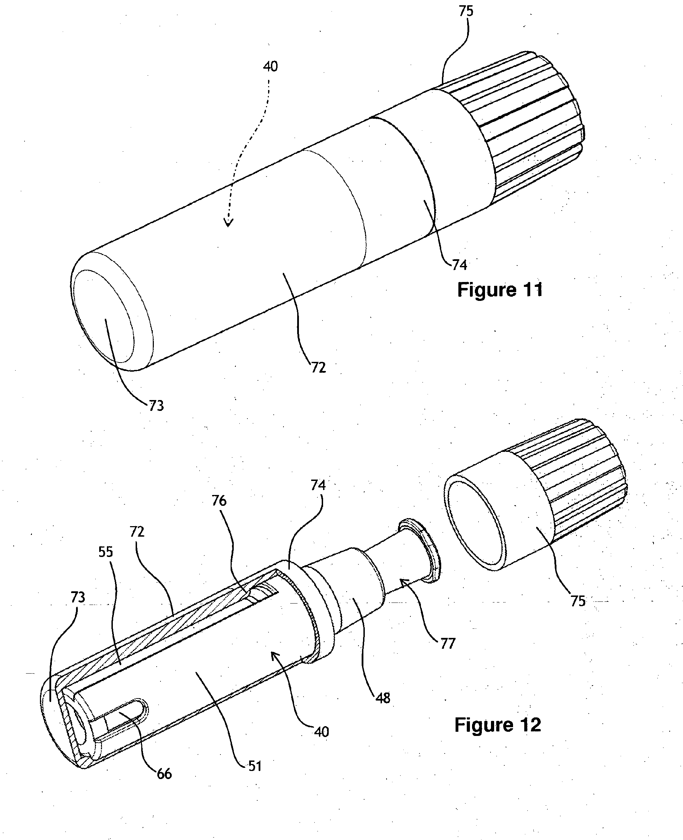

Figures 11 and 12 show a package including a safety device 40 essentially corresponding to that described with reference to Figures 4 to 8, the package including a tubular component 72 closed at one end 73 and open at the other end 74, for receiving the safety device. A cap 75 fits to the open other end 74 of the tubular component and may be provided with a tamper-evident seal (not shown) such as an adhesive paper strip bridging the joint between the tubular component 72 and the cap 75. The bore within the component 72 includes opposed ribs 76 which overlie the fingers 55 of the device located within the bore, to prevent activation of the device (i.e. forward movement of the control member 60 relative to the fingers 55) by bearing on those fingers and preventing radially outward movement thereof.

The device is pre-fitted with a conventional needle having a hub 77 which has a bore forming a part of a conventional syringe connector, such as a Luer slip or Luer lock connector. The hub may fit into the mount 48 of the device, as has been described above with reference to Figures 4 to 8. In the alternative, the hub could be specially configured so as to serve as the mount for the device. Sterility may be maintained by fitting the device into the tubular component 72 and closing the other end 74 with cap 75 within a sterile environment, and then fitting a seal around the junction between the cap and tubular component. That seal may also serve to give tamper-evidence.

The package of Figures 11 and 12 is used by twisting the cap 75 off the tubular component 72, breaking the seal therebetween if one is provided, and then a conventional syringe is connected to the exposed hub 77 of the needle. Once fully connected, the syringe is used to pull the device 40 out of the tubular component 72 whereafter the device is set ready for use as has been described in connection with Figures 4 to 8.

The arrangement of Figures 11 and 12 is equally applicable to the second safety device of Figures 9 and 10. Moreover, by making the tubular component 72 and cap 75 of a sufficient internal length, when fitted together, the tubular component may accommodate a pre-filled syringe having a safety device conferring protection to the needle of that safety device. In such a case, the tubular component 72 may be furnished with an internal structure for stripping a soft needle cover from the needle, as has been described with Figures 1 to 3.

## Claims

1. A medical needle safety device (24) having a mount (21) for a medical needle (23), a sleeve (25) for shielding a mounted needle and movable from an initial shielding position to a non-shielding position, a finger (28) provided on one of the sleeve and mount and resiliently deformable outwardly by movement of the sleeve (25) from its initial shielding position to a non-shielding position, and a package component (35) for the safety device which overlies at least a part of the finger (28), **characterised in that** resilient outward deformation of the finger (28) stores energy therein for returning the sleeve (25) to the needle shielding position, and the package component prevents outward deformation of the finger to block movement of the sleeve until the package component (35) is removed from the safety device (24).

2. A medical needle safety device as claimed in claim 1, wherein:
- the sleeve (25) is slidably arranged coaxially with the mount (21) so that a force applied to the sleeve moves the sleeve from an initial needle shielding position to a non-shielding position whereat at least the tip of a supported needle (23) is exposed beyond the sleeve;
- an abutment surface (31;65) and a sliding surface (57) is provided on the other of the sleeve and mount; and
- the finger (28) has a part (32) in radial alignment with the abutment surface to block movement of the sleeve from its needle shielding position.

3. A medical needle safety device as claimed in claim 2, wherein there is a control member (26) arranged coaxially with the sleeve (25) and mount and slidably displaceable from a set position with respect thereto.

4. A medical needle safety device as claimed in any of claims 1 to 3, wherein the package component 35) is substantially tubular and closely surrounds the sleeve (25) to prevent outward deformation of the finger (28).

5. A medical needle safety device as claimed in any of claims 1 to 3, wherein the package component (35) is substantially tubular and is provided with an internal rib (76) which overlies the finger to prevent the outward deformation thereof.

6. A medical needle safety device as claimed in claim 4 or claim 5, wherein the package component (72) has a closed end (73) and an open end (74), the safety device being insertable into the package component (72) and removable therefrom through the open end (74) of the component.

7. A medical needle safety device as claimed in claim 6, wherein a sealing member is arranged to close the open end (74) of the package component (72) and so enclose the safety device therewithin.

8. A medical needle safety device as claimed in claim 6, wherein there is a removable cap (75) for the open end (74) of the package component (72).

9. A medical needle safety device as claimed in claim 8 in combination with a medical needle (41) having a hub (46) received in the mount, and the sleeve (51) being disposed in a shielding position with respect to the needle.

10. A medical needle safety device as claimed in claim 8 in combination with a single-use syringe (21) having a needle (23) permanently attached thereto, the syringe (21,22) serving as the needle mount of the safety device and the sleeve (25) is arranged to slide on the syringe.

11. A medical needle safety device as claimed in claim 10 and in which a needle cover (34) is provided for the needle (23) of the single-use syringe (21), said cover sealing the sharp tip of the needle.

12. A medical needle safety device as claimed in claim 11, wherein the package component (35) includes a structure (38) for stripping the needle cover (34) from the needle (23) on removing the package component (35) from the safety device.

13. A medical needle safety device as claimed in any of the preceding claims, wherein after the return of the sleeve (35) to a needle shielding position, the finger (28) serves to block subsequent axial sliding movement of the sleeve (25) to a non-shielding position.

14. A medical needle safety device as claimed in any of the preceding claims, wherein there is a plurality of fingers (28) spaced around said one of the sleeve (25;70) and mount (22;69), the fingers being resiliently deformable outwardly by sliding axial movement of the sleeve (25).

## Patentansprüche

1. Medizinische Nadelschutzvorrichtung (24) mit einer Befestigung (21) für eine medizinische Nadel (23), einer von einer ursprünglichen Abschirmposition in eine Nicht-Abschirmposition bewegbaren Hülse (25) zum Abschirmen einer befestigten Nadel, einem an der Hülse oder der Befestigung vorgesehenen Finger (28), der bei einer Bewegung der Hülse (25) aus ihrer ursprünglichen Abschirmposition in die Nicht-Abschirmposition elastisch nach Außen verformbar ist, und einer Verpackungskomponente (35) für die Schutzvorrichtung, die mindestens einen Teil des Fingers (28) überdeckt, **dadurch gekennzeichnet, dass** die elastische Verformung des Fingers (28) nach Außen Energie zum Zurückführen der Hülse (25) in die Nadel-Abschirmposition speichert, und die Verpackungskomponente eine Verformung des Fingers nach Außen verhindert, um eine Bewegung der Hülse zu blockieren, bis die Verpackungskomponente (35) von der Schutzvorrichtung (24) entfernt ist.

2. Medizinische Nadelschutzvorrichtung nach Anspruch 1, wobei:
- die Hülse (25) verschiebbar und koaxial zu der Befestigung (21) angeordnet ist, so dass eine auf die Hülse wirkende Kraft die Hülse aus der ursprünglichen Nadel-Abschirmposition in die Nicht-Abschirmposition bewegt, in der sich mindestens eine Spitze der gehaltenen Nadel (23) über die Hülse hinaus erstreckt;
- eine Anlagefläche (31; 65) und eine Gleitfläche (57) auf der Hülse oder der Befestigung vorgesehen ist; und
- der Finger (28) einen Teil (32) in radialer Ausrichtung mit der Anlagefläche hat, um eine Bewegung der Hülse aus ihrer Nadel-Abschirmposition zu blockieren.

3. Medizinische Nadelschutzvorrichtung nach Anspruch 2, wobei ein Kontrollelement (26) koaxial zu der Hülse (25) und der Befestigung angeordnet und verschieblich aus einer vorgegebenen Position in Bezug zu dieser versetzbar ist.

4. Medizinische Nadelschutzvorrichtung nach einem der vorhergehenden Ansprüche 1 bis 3, wobei die Verpackungskomponente (35) im Wesentlichen rohrförmig ist und die Hülse (25) eng umschließt, um eine Verformung des Fingers (28) nach Außen zu verhindern.

5. Medizinische Nadelschutzvorrichtung nach einem der vorhergehenden Ansprüche 1 bis 5, wobei die Verpackungskomponente (35) im Wesentlichen rohrförmig ist und eine Innenrippe (76) aufweist, die den Finger überlagert, um eine Verformung des Fingers nach Außen zu verhindern.

6. Medizinische Nadelschutzvorrichtung nach Anspruch 4 oder Anspruch 5, wobei die Verpackungskomponente (72) ein geschlossenes Ende (73) und ein offenes Ende (74) hat, die Schutzvorrichtung ist in die Verpackungskomponente (72) einführbar und aus dieser durch das offene Ende (74) der Komponente ausführbar.

7. Medizinische Nadelschutzvorrichtung nach Anspruch 6, wobei ein Verschlusselement zum Verschließen des offenen Endes (74) der Verpackungskomponente (72) vorgesehen ist, um somit die Schutzvorrichtung darin einzuschließen.

8. Medizinische Nadelschutzvorrichtung nach Anspruch 6, wobei eine entfernbare Kappe (75) für das offene Ende (74) der Verpackungskomponente (72) vorhanden ist.

9. Medizinische Nadelschutzvorrichtung nach Anspruch 8 in Kombination mit einer medizinischen Nadel (41) mit einem in der Befestigung aufgenommenen Ansatz (46), wobei die Hülse (51) in einer Abschirmposition in Bezug zu der Nadel verlagert ist.

10. Medizinische Nadelschutzvorrichtung nach Anspruch 8 in Kombination mit einer Einweg-Spritze (21) mit einer dauerhaft daran befestigten Nadel (23), die Spritze (21, 22) dient als die Nadelbefestigung der Schutzvorrichtung und die Hülse (25) ist zum Verschieben an der Spritze angeordnet.

11. Medizinische Nadelschutzvorrichtung nach Anspruch 10, bei der eine Nadelabdeckung (34) für die Nadel (23) der Einweg-Spritze (21) vorgesehen ist, die Abdeckung deckt die scharfe Spitze der Nadel ab.

12. Medizinische Nadelschutzvorrichtung nach Anspruch 11, wobei die Verpackungskomponente (35) eine Struktur (38) zum Abziehen der Nadelabdeckung (34) von der Nadel (23) beim Entfernen der Verpackungskomponente (35) von der Schutzvorrichtung aufweist.

13. Medizinische Nadelschutzvorrichtung nach einem der vorhergehenden Ansprüche, wobei nach der Rückkehr der Hülse (35) in eine Nadel-Abschirmposition der Finger (28) zum Blockieren einer nachfolgenden axialen Verschiebebewegung der Hülse (25) in eine Nicht-Abschirmposition dient.

14. Medizinische Nadelschutzvorrichtung nach einem der vorhergehenden Ansprüche, wobei eine Mehrzahl von Fingern (28) um die Hülse (25; 70) oder die Befestigung (22; 69) verteilt ist, die Finger sind bei einer axialen Verschiebebewegung der Hülse (25) elastisch nach Außen verformbar.

## Revendications

1. Un dispositif de sécurité (24) pour aiguille médicale possédant une monture (21) pour une aiguille médicale (23), un manchon (25) destiné à protéger une aiguille montée et pouvant se déplacer d'une position initiale de protection vers une position de non protection, un doigt (28) pourvu sur le manchon ou sur la monture et déformable élastiquement vers l'extérieur par le mouvement du manchon (25) de sa position initiale de protection vers une position de non protection, et un élément d'emballage (35) pour le dispositif de sécurité qui recouvre au moins une partie du doigt (28), **caractérisé en ce que** ladite déformation élastique vers l'extérieur du doigt (28) emmagasine de l'énergie dans celui-ci afin d'assurer le mouvement retour du manchon (25) vers la position de protection de l'aiguille, et l'élément d'emballage empêche la déformation vers l'extérieur du doigt afin de bloquer le mouvement du manchon jusqu'à ce que l'élément d'emballage (35) soit retiré du dispositif de sécurité (24).

2. Un dispositif de sécurité pour aiguille médicale tel que revendiqué dans la revendication 1, dans lequel:
- le manchon (25) est agencé de façon coulissante coaxialement à la monture (21) de façon à ce qu'une force appliquée au manchon déplace ledit manchon d'une position initiale de protection d'aiguille vers une position de non protection où au moins la pointe d'une aiguille (23) maintenue dans le dispositif est exposé au-delà du manchon ;
- une surface de butée (31 ; 65) et une surface de glissement (57) sont aménagées sur l'autre partie, manchon ou monture ; et
- le doigt (28) possède une partie (32) en alignement radial avec la surface de butée afin de bloquer le mouvement du manchon de sa position de protection de l'aiguille.

3. Un dispositif de sécurité pour aiguille médicale tel que revendiqué dans la revendication 2, dans lequel il existe un élément de commande (26) disposé coaxialement avec le manchon (25) et la monture et pouvant être déplacé de façon coulissante d'une position réglée par rapport à ceux-ci.

4. Un dispositif de sécurité pour aiguille médicale selon l'une des revendications 1 à 3 dans lequel l'élément d'emballage (35) est de forme sensiblement tubulaire et entoure étroitement le manchon (25) de façon à empêcher la déformation vers l'extérieur du doigt (28).

5. Un dispositif de sécurité pour aiguille médicale selon l'une des revendications 1 à 3 dans lequel l'élément emballage (35) est de forme sensiblement tubulaire et est pourvu d'une nervure interne (76) qui recouvre le doigt de manière à empêcher la déformation vers l'extérieur de celui-ci.

6. Un dispositif de sécurité pour aiguille médicale tel que revendiqué dans la revendication 4 ou la revendication 5, dans lequel l'élément d'emballage (72) possède une extrémité fermée (73) et une extrémité ouverte (74), le dispositif de sécurité pouvant être inséré dans l'élément d'emballage (72) et retiré de celui-ci à travers l'extrémité ouverte (74) dudit composant.

7. Un dispositif de sécurité pour aiguille médicale tel que revendiqué dans la revendication 6, dans lequel un élément d'étanchéité est agencé pour fermer l'extrémité ouverte de l'élément d'emballage (72) et ainsi renfermer le dispositif à l'intérieur de celui-ci.

8. Un dispositif de sécurité pour aiguille médicale tel que revendiqué dans la revendication 6, dans lequel il existe un capuchon amovible (75) pour l'extrémité ouverte (74) de l'élément d'emballage (72).

9. Un dispositif de sécurité pour aiguille médicale tel que revendiqué dans la revendication 8 en combinaison avec une aiguille médicale (41) possédant un moyeu (46) reçu dans la monture, le manchon (51) étant disposé dans une position de protection par rapport à l'aiguille.

10. Un dispositif de sécurité pour aiguille médicale tel que revendiqué dans la revendication 8 en combinaison avec une seringue à usage unique (21) possédant une aiguille (23) qui y est attachée de façon permanente, ladite seringue (21, 22) servant comme la monture d'aiguille du dispositif de sécurité, le manchon étant agencé pour glisser sur la seringue.

11. Un dispositif de sécurité pour aiguille médicale tel que revendiqué dans la revendication 10 et dans lequel il existe un dispositif de recouvrement d'aiguille pour l'aiguille (23) de la seringue à usage unique (21), ledit dispositif de recouvrement d'aiguille renfermant la pointe acérée de l'aiguille.

12. Un dispositif de sécurité pour aiguille médicale tel que revendiqué dans la revendication 11 dans lequel l'élément d'emballage (35) comporte une structure (38) destinée à arracher le dispositif de recouvrement d'aiguille (34) de l'aiguille (23) lorsque l'élément d'emballage (35) est retiré du dispositif de sécurité.

13. Un dispositif de sécurité pour aiguille médicale selon l'une quelconque des revendications précédentes, dans lequel, après le retour du manchon (35) dans la position de protection de l'aiguille, le doigt (28) sert à bloquer le mouvement de coulissement axial ultérieur du manchon (25) vers une position de non protection.

14. Un dispositif de sécurité pour aiguille médicale selon l'une quelconque des revendications précédentes, dans lequel il existe une pluralité de doigts (28) espacés autour dudit manchon (25 ; 70) ou de ladite monture (22 ; 69), lesdits doigts étant déformables élastiquement vers l'extérieur par le mouvement de coulissement axial du manchon (25).
